# EUROPEAN PATENT SPECIFICATION

(11) **EP 2 164 529 B1**
(45) Date of publication and mention of the grant of the patent: **27.06.2012**
(21) Application number: 08785886.6
(22) Date of filing: 27.06.2008
(51) Int. Cl.: A61L 9/03, A61L 9/12, A01M 1/00

(54) **CONTAINER FOR VOLATILE SUBSTANCES WITH LEVEL INDICATOR LIQUID**
BEHÄLTER FÜR FLÜCHTIGE SUBSTANZEN MIT FÜLLSTANDSANZEIGEFLÜSSIGKEIT
CONTENANT POUR SUBSTANCES VOLATILES AVEC INDICATEUR DE NIVEAU LIQUIDE

(30) Priority: 29.06.2007 US 947048 P
(43) Date of publication of application: 24.03.2010
(73) Proprietor: Zobele Holding SpA, 38100 Trento (IT)
(72) Inventor: DEFLORIAN, Stefano, I-38100 Trento (IT); PEDROTTI, Andrea, I-38100 Trento (IT)
(74) Representative: Carpintero Lopez, Francisco
(86) International application number: PCT/EP2008/058286
(87) International publication number: WO 2009/003947

(56) References cited:
- US-A- 4 526 320
- US-A1- 2004 045 495
- US-A1- 2006 231 641
- US-B1- 6 569 387

## Description

### OBJECT OF THE INVENTION

The present invention refers to a container of volatile substances such as insecticides and/or perfumes. The second volatile substance is used as a visual indication of the end of life of the first substance.

### BACKGROUND ART

Some of the prior-art evaporation devices use a porous solid material impregnated with a volatile substance, from which said substance is evaporated. The drawback associated to these devices, is that it is not possible to detect visually when the volatile product has been consumed because the appearance of the porous material is not altered.

There are known devices that in part solve this problem, because these devices include a first container from which a volatile substance is evaporated, and a second container with a typically coloured liquid substance, that serves to indicate visually when the volatile substance has been consumed. These devices have been designed so that the volatile substance and the coloured liquid, are completely evaporated more or less at the same time, even if they are under different temperature conditions.

However, this solution is not satisfactory because it requires the manufacture of two different containers, that need to be assembled together during the manufacturing process of the device, for that the cost of the device is increased.

Additionally, it is difficult to arrange the two containers at the same distance of the heating means of an evaporation device, thus it results that the evaporation of the volatile substance and the indicative liquid is not synchronized.

The following patients belong to the technical filed of this invention US 2006/0231641, US-6.569.387, US-4.526.320 and US 2004/0045495.

### DESCRIPTION OF THE INVENTION

The invention is defined by the appended claims. The container of the invention has two chambers for containing respectively a porous carrier impregnated with a first volatile substance, and a second chamber containing a second volatile substance. In the present invention porous carrier refers to any type of porous material, including porous plastics.

The present invention solves the drawbacks of prior-art techniques, because it consist of a single container having two separate chambers, one for the solid carrier of the volatile substance, and the other for containing an indicative liquid which evaporates through a permeable film.

The indicative liquid may be coloured, so that for the user it may be easier to visualize the status of the volatile substance.

The container, the amount of volatile substance and indicative liquid, and the composition of both, are designed so that the rate of evaporation is substantially the same for both elements. The skilled person in the art using his common general knowledge, knows how to design the container and select a volatile substance and a liquid, in order to synchronise the evaporation of both elements.

Another aspect of the invention refers to an electric evaporation device which includes the contained previously described. Since a single container includes the two chambers, it is possible to design the container in such a manner that both chambers are located at a similar or suitable distance from the heating means of the device, so that it is possible to synchronise the rate of evaporation of the volatile substance and the indicative liquid. This arrangement has been represented in the attached figures.

Additionally, the invention provides the advantage that for the users is more convenient to replace only one container when the volatile substance has been consumed, than the two containers used by prior-art devices.

The container may be thermoformed using a transparent plastic plate.

### DESCRIPTION OF THE DRAWINGS

To complement the description being made and with the object of aiding towards a better understanding of the characteristics of the invention, in accordance with a preferred example of embodiment thereof, a set of drawings is attached as an integral part of said description, wherein the following has been represented, with an illustrative, non-limiting character:
Figure 1.- shows an exploded perspective view of the container of the invention.
Figure 2.- shows another exploded perspective view of the electric device of the invention.

### PREFERRED EMBODIMENT OF THE INVENTION

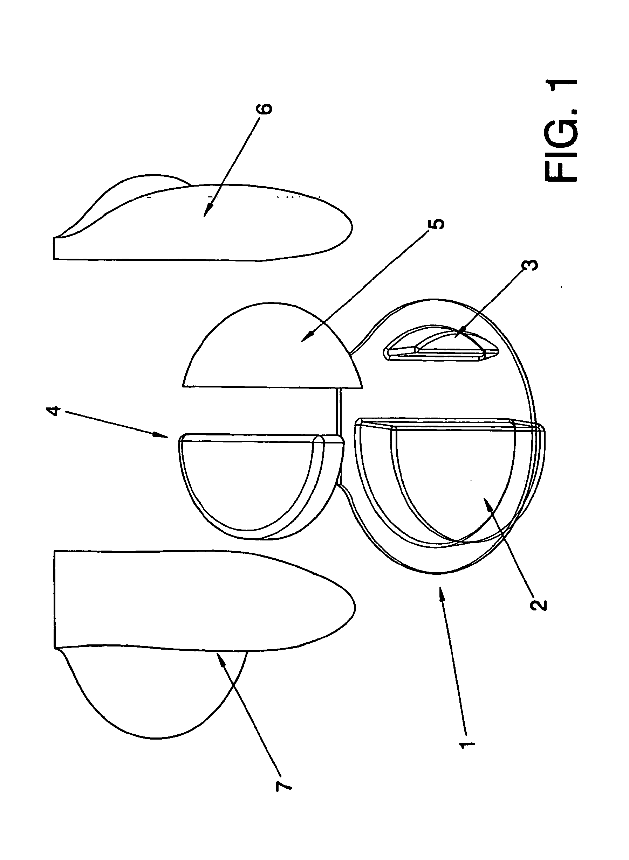

In figure 1 it can be observed that the container (1) of the invention has a first chamber (2) containing a porous material (4) impregnated with a first volatile substance, and a second chamber (3) containing a second volatile substance.

The container is configured to allow the evaporation of said volatile substances. This is achieved by an opening in said first chamber by means of which the porous material is in contact with the air. Similarly the second chamber has an opening closed by a permeable film (5) which allows the evaporation of the second volatile substance in a vapour state.

The second volatile substance is a liquid or a gel.

A first and a second peealable films (6,7) are respectively provided closing said first and second chamber during storage and transport of the container.

The evaporation of the first and the second volatile substances is synchronized so that both volatile substances are finished (consumed) substantially at the same time. In other words, the rate of evaporation of the first and the second substances has been selected so that both volatile substances are finished substantially at the same time. This is achieved by selecting a proper volatile substances and permeable film, and properly configuring the container.

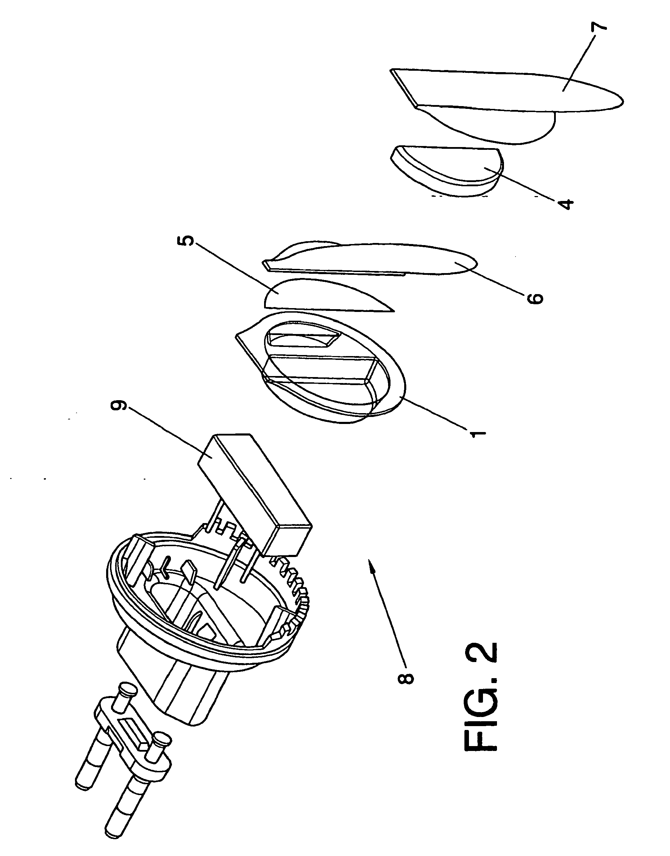

Figure 2 shows an electric device (8) for the evaporation of volatile substances which includes the previously-described container. The device comprises heating means (9) arranged to heat said first and second volatile substances.

## Claims

1. Container (1) configured to allows the evaporation of volatile substances, comprising a first and a second chambers (2,3), wherein said first chamber (2) contains a porous material (4) impregnated with a first volatile substance which contains an insecticide and/or a perfume, and wherein the second chamber (3) contains an indicative volatile substance suitable for providing a visual indication of the end of life of the first volatile substance, **characterised in that** said second chamber is closed by a permeable film (5), which allows the evaporation of the indicative volatile substance in a vapour state, and wherein the amount of said first and indicative volatile substances and their composition, are selected to synchronize the evaporation of said volatile substances, so that the evaporation of said volatile substances is synchronized so that both volatile substances are finished at the same time.

2. Container according to claim 1 wherein the container comprises a transparent plastic plate in which said first and a second chambers (2,3) are formed.

3. Container according to claim 2 wherein the container has been thermoformed using said transparent plastic plate.

4. Container according to claim 1 wherein said first chamber is open to the air.

5. Container according to claim 1 wherein the first and the second chambers are closed by a peealable films (6,7) provided for closing the first and second chamber during storage and transport of the container.

6. Container according to claim 1 wherein the indicative volatile substance is coloured.

7. Container according to any of the preceding claims wherein the indicative volatile substance is a liquid or a gel.

8. Device (8) for the evaporation of volatile substances including a container according to any of the preceding claims, and heating means (9) arranged to heat said first and said indicative volatile substances.

## Patentansprüche

1. Behälter (1), der zum Gestatten der Verdunstung flüchtiger Substanzen ausgebildet ist, mit einer ersten und einer zweiten Kammer (2, 3), wobei die erste Kammer (2) ein poröses Material (4) aufweist, welches mit einer ersten flüchtigen Substanz getränkt ist, die ein InseiCtizid und/oder ein Parfum enthält, und wobei die zweite Kammer (3) eine anzeigende flüchtige Substanz aufweist, die für das Bereitstellen einer visuellen Anzeige des Endes der Lebensdauer der ersten flüchtigen Substanz geeignet ist, dadurch gekenntzeichnet, dass die zweite Kammer (3) durch eine permeable Schicht (5) verschlossen ist, die die Verdunstung der anzeigenden flüchtigen Substanz in einen gasförmige Zustand gestattet, und wobei die Menge der ersten und der anzeigenden flüchtigen Substanzen und ihre Zusammensetzung ausgewählt sind, um die Verdunstung der flüchtige Substanzen zu synchronisieren, so dass die Verdunstung der flüchtigen Substanzen so synchronisiert ist, dass beide flüchtigen Substanzen zum gleichen Zeitpunkt aufgebraucht sind.

2. Behälter (1) nach Anspruch 1, wobei der Behälter (1) eine transparente Kunststoffplatte aufweist, in der die erste und zweite Kammer (2, 3) ausgebildet sind.

3. Behälter (1) nach Anspruch 2, wobei der Behälter (1) unter Verwendung der transparenten Kunststoffplatte warmgeformt wurde.

4. Behälter (1) nach Anspruch 1, wobei die erste Kammer (2) zur Luft hin offen ist.

5. Behälter (1) nach Anspruch 1, wobei die erste und die zweite Kammer (2, 3) durch abziehbare Schichten (6, 7) verschlossen sind, die zum Verschließen der ersten und zweiten Kammer (2, 3) während der Lagerung und des Transports des Behälters (1) bereitgestellt sind.

6. Behälter (1) nach Anspruch 1, wobei die anzeigende flüchtige Substanz farbig ist.

7. Behälter (1) nach einem der vorangehenden Ansprüche, wobei die anzeigende flüchtige Substanz eine Flüssigkeit oder ein Gel ist.

8. Vorrichtung (8) für die Verdunstung flüchtiger Substanzen, mit einem Behälter (1) nach einem der vorangehenden Ansprüche und Heizmitteln (9), die zum Heizen der ersten und der anzeigenden flüchtige Substanzen angeordnet sind.

## Revendications

1. Récipient (1) configuré de manière à permettre l'évaporation de substances volatiles, comprenant une première et une deuxième chambres (2,3), dans lequel ladite première chambre (2) contient un matériau poreux (4) imprégné d'une première substance volatile qui contient un insecticide et/ou un parfum, et dans lequel la deuxième chambre (3) contient une substance volatile indicatrice adéquate pour fournir une indication visuelle de la fin de vie de la première substance volatile, **caractérisé en ce que** ladite deuxième chambre est fermée par un film perméable (5) qui permet l'évaporation de la substance volatile indicatrice à l'état de vapeur, et dans lequel la quantité de ladite première substance volatile et de ladite substance volatile indicatrice et leur composition sont choisies de manière à synchroniser l'évaporation desdites substances volatiles, de sorte que l'évaporation desdites substances volatiles soit synchronisée de manière à ce que les deux substances volatiles s'épuisent en même temps.

2. Récipient selon la revendication 1, dans lequel le récipient comprend une plaque en plastique transparent dans laquelle ladite première chambre et une deuxième chambres (2,3) sont formées.

3. Récipient selon la revendication 2, dans lequel le récipient a été thermoformé à l'aide de ladite plaque en plastique transparent.

4. Récipient selon la revendication 1, dans lequel ladite première chambre est ouverte à l'air.

5. Récipient selon la revendication 1, dans lequel la première et la deuxième chambres sont fermées par un film détachable (6,7) destiné à fermer la première et la deuxième chambres pendant le stockage et le transport du récipient.

6. Récipient selon la revendication 1, dans lequel la substance volatile indicatrice est colorée.

7. Récipient selon l'une quelconque des revendications précédentes, dans lequel la substance volatile indicatrice est un liquide ou un gel.

8. Dispositif (8) pour l'évaporation de substances volatiles comprenant un récipient selon l'une quelconque des revendications précédentes, et des moyens de chauffage (9) conçus pour chauffer ladite première substance volatile et ladite substance volatile indicatrice.
